# DEMANDE DE BREVET EUROPEEN

(11) **EP 4 067 498 A1**
(43) Date de publication de la demande: **05.10.2022**
(21) Numéro de dépôt: 22163703.6
(22) Date de dépôt: 23.03.2022
(51) Int. Cl.: C12P 19/02, C12P 19/12, C12P 19/14, C12P 7/06, C08H 8/00, C13B 10/00, C13B 20/00

(54) **PROCÉDÉ DE TRAITEMENT DE BIOMASSE LIGNO-CELLULOSIQUE**

(30) Priorité: 30.03.2021 FR 2103229
(71) Demandeur: IFP Energies nouvelles, 92500 Rueil-Malmaison (FR)
(72) Inventeur: COUDERC, Sophie, 92852 RUEIL-MALMAISON CEDEX (FR); JACQUIN, Marc, 92852 RUEIL-MALMAISON CEDEX (FR); AYMARD, Caroline, 92852 RUEIL-MALMAISON CEDEX (FR)
(74) Mandataire: IFP Energies nouvelles

(57) **Abrégé**

La présente invention concerne un procédé de traitement d'une biomasse lignocellulosique préalablement prétraitée (1), ledit procédé comprenant l'extraction des sucres libres de ladite biomasse prétraitée par une solution d'extraction alcoolique ou hydro-alcoolique (2), de façon à obtenir une phase liquide enrichie en sucres libres, appelée jus (3), et une phase solide appauvrie en sucres libres, appelée marc (4).

## Description

### Domaine technique

L'invention concerne un procédé de traitement de biomasse lignocellulosique pour produire des jus sucrés dits de seconde génération (2G). Ces jus sucrés peuvent être utilisés pour produire d'autres produits par voie chimique ou voie biochimique/fermentaire (par exemple, des alcools comme l'éthanol, le butanol ou d'autres molécules, par exemple des solvants tels que l'acétone, le furfural, le xylitol, et d'autres molécules biosourcées...).

### Technique antérieure

La biomasse lignocellulosique représente une des ressources renouvelables les plus abondantes sur terre. Les substrats considérés sont très variés, ils concernent à la fois des substrats ligneux comme différents bois (feuillus et résineux), des coproduits issus de l'agriculture (pailles de blé, pailles de riz, rafles de maïs, bagasse, etc...) ou encore industries papetières, etc... La biomasse lignocellulosique est composée de trois principaux polymères : la cellulose (35 à 50%), qui est un polysaccharide cristallin essentiellement constitué de glucose ; l'hémicellulose (20 à 30%), qui est un polysaccharide amorphe constitué principalement de xylose, galactose, arabinose, mannose, glucose; et de la lignine (15 à 25%), qui est un polymère de structure complexe et de haut poids moléculaire, composé de différents monomères phénoliques reliés par des liaisons éther. Ces différents polymères sont responsables des propriétés intrinsèques de la paroi végétale et s'organisent en un enchevêtrement complexe. Parmi les trois polymères de base qui intègrent la biomasse lignocellulosique, la cellulose et l'hémicellulose sont ceux qui permettent la production de jus sucrés dits de deuxième génération (2G) et d'éthanol 2G. Il faut noter que la composition des hémicelluloses est variable en fonction de la nature de la biomasse lignocellulosique considérée.

Il existe de nombreux procédés de valorisation de la biomasse lignocellulosique en sucres 2G ou éthanol 2G, qui diffèrent par les conditions opératoires appliquées et la nature des réactifs utilisés pour libérer les sucres. Néanmoins, les procédés de valorisation de la biomasse lignocellulosique en sucres 2G les plus développés à l'échelle industrielle ont pour point commun notamment une étape de prétraitement et une étape d'hydrolyse enzymatique par un cocktail enzymatique. L'étape de prétraitement permet de déstructurer la biomasse native, et généralement une partie du polymère hémicellulose est dépolymérisée à cette étape. L'hydrolyse enzymatique cible principalement la cellulose, et les hémicelluloses résiduels, les polymères sont dépolymérisés sous l'action d'un cocktail d'enzymes.

Ces jus de sucres 2G peuvent être utilisés/valorisés par exemple dans un procédé fermentaire ou chimique, tels quels ou éventuellement être traités ultérieurement. Le plus souvent, le procédé global comprend des étapes de séparation intermédiaires et/ou une étape de purification du ou des produits finaux.

Le prétraitement permet de modifier les propriétés physico-chimiques de la biomasse lignocellulosique afin de rendre accessible la cellulose aux enzymes et atteindre une bonne réactivité en hydrolyse enzymatique. De nombreuses technologies de prétraitement existent et permettent la mise en température de la biomasse dans des conditions chimiques variées. Le prétraitement peut être effectué avec ou sans ajouts de produits acides ou basiques. Il peut aussi être effectué dans un solvant comme l'eau ou un produit organique comme, par exemple, de l'alcool (procédé dit organosolv) mais aussi dans un milieu peu diluant comme de la vapeur. Ce prétraitement peut aussi comporter une étape physique comme un défibrage ou une détente explosive dans le cadre d'une explosion à la vapeur. Ce prétraitement peut aussi avoir plusieurs étapes permettant d'optimiser le procédé global, comme par exemple, une cuisson acide suivie d'une explosion à la vapeur ou deux explosions à la vapeur consécutives. Les prétraitements ci-après regroupés sous le terme générique de « cuisson » concernent des traitements thermiques, avec des températures supérieures à 100°C. Pour des raisons technologiques notamment de mélange et de pompabilité, les cuissons sont réalisées en conditions dites diluées avec des ratios massiques de liquide de cuisson / biomasse supérieurs à 2. Les cuissons couplent généralement un effet thermique à un effet chimique obtenu par des pH bas ou au contraire élevé et/ou la mise en oeuvre de solvants. Ce terme de cuisson regroupe par exemple les cuissons acides, les cuissons alcalines, les cuissons « organosolv». Ce dernier procédé concerne un prétraitement en présence d'un ou plusieurs solvants organiques et généralement d'eau. Le solvant peut être de manière non limitative un alcool (éthanol), un acide type acide acétique, acide formique, ou encore l'acétone. Les procédés « organosolv pulping » conduisent à une solubilisation au moins partielle de la lignine, une solubilisation partielle des hémicelluloses. On a alors deux flux en sortie : le substrat prétraité avec cellulose, hémicellulose et lignine résiduelles et la phase solvant qui contient une partie de la lignine et une partie des hémicelluloses solubilisées. Il y a généralement une étape de régénération du solvant qui permet d'extraire un flux de lignine solide. Dans certains prétraitements, dits « organosolv pulping » (notamment avec de l'éthanol), l'action du solvant organique est améliorée par l'ajout d'un acide fort (du type H₂SO₄). On peut alors envisager d'avoir une mise en contact de la biomasse avec le solvant via un réacteur d'imprégnation avant la phase de cuisson et/ou d'avoir une mise en contact de la biomasse avec le catalyseur acide avant d'effectuer une cuisson « organosolv pulping ».

Une solution technologique est bien connue de l'homme de l'art pour pallier les problèmes technologiques de la cuisson : Les réacteurs mettant en oeuvre une détente explosive comme mode de sortie de la biomasse prétraitée (encore nommée explosion à la vapeur ou Steam gun ou Steam explosion selon la terminologie anglo-saxonne), permettent de s'affranchir des problèmes de pompabilité en sortie de cuisson. Le prétraitement de type explosion à la vapeur permet de travailler avec des ratios massiques de liquide / biomasse inférieurs à 2, la biomasse est ainsi soumise à une quantité proportionnellement faible de vapeur d'eau. Une partie de la vapeur d'eau servant à l'entrainement du solide au cours de la détente explosive peut être ensuite séparée du milieu. La détente explosive permet ainsi de produire un marc prétraité ne contenant pas de phase liquide continue, on peut le qualifier de « sec » par opposition aux marcs produits par des cuissons conventionnelles, bien que sa teneur en eau soit généralement supérieure à 30% poids. Le plus souvent, l'explosion à la vapeur en condition acide est préférée, car elle permet un bon compromis entre l'hydrolyse acide de l'hémicellulose et la réactivité de la cellulose en hydrolyse enzymatique, avec une hydrolyse presque totale de l'hémicellulose et une amélioration importante de l'accessibilité et la réactivité de la cellulose aux enzymes. Ce prétraitement peut être précédé d'autre(s) traitement(s) (broyage, imprégnation, cuisson etc...).

On note que ce type de prétraitement, cuisson ou explosion à la vapeur, requiert une transformation de la biomasse brute en un substrat prétraité réactif, avant de démarrer les étapes suivantes de conversion de la cellulose, qui est le polymère le plus réfractaire. Après ce prétraitement, des sucres (sucres en C5 et C6) se retrouvent libérés sous forme monomérique ou oligomérique solubles dans l'eau. L'extraction de ces sucres libres dans un jus est intéressante pour une valorisation dans le reste du procédé de transformation de la biomasse ou dans un autre procédé (en parallèle) ou pour une commercialisation telle quelle du jus sucré. Il y a donc un intérêt à extraire ces jus de sucres libres du substrat prétraité, avant que le marc prétraité en question, qui sera alors enrichi en matière solide et appauvri en sucres, ne subisse les traitements suivants de conversion de la cellulose. Dans cette optique, dans le cas d'un prétraitement de type cuisson, la fraction liquide et la fraction solide de la biomasse prétraitée sont séparées, puis la fraction solide est éventuellement lavée à l'eau pour récupérer davantage de sucres. En revanche, dans le cas d'un prétraitement de type explosion à la vapeur, on récupère en fin de prétraitement un marc à haute teneur en matière sèche (typiquement supérieur à 40% poids). L'extraction des sucres libres nécessite au préalable une étape dite de « repulpage » du marc dans l'eau telle que décrite dans le brevet EP-3 587 583, avant de séparer la fraction liquide et la fraction solide, puis de réaliser un éventuel lavage à l'eau de la fraction solide pour récupérer davantage de sucres libres.

L'étape de cuisson et/ou d'explosion à la vapeur est souvent précédée d'une étape d'imprégnation. Elle est souvent suivie d'une étape d'hydrolyse enzymatique de la cellulose utilisant un cocktail enzymatique dédié. Ce cocktail enzymatique est une solution contenant des enzymes ayant des activités cellulolytiques et/ou hémicellulolytiques qui vont hydrolyser de manière sélective le polymère de cellulose et éventuellement les polymères résiduels d'hémicelluloses. De par l'action ciblée des enzymes, la lignine résiduelle est peu affectée par l'hydrolyse enzymatique. L'hydrolyse enzymatique se conduit dans des conditions douces de températures et de pH. Typiquement, à une température de 30°C à 55°C, et un pH entre 3 et 7. La réaction d'hydrolyse enzymatique des polymères de sucres en monomères nécessite l'ajout de molécules d'eau, l'hydrolyse étant favorisée par la présence d'un milieu liquide continu. Il existe de multiples technologies de réacteurs permettant sa mise en œuvre. L'hydrolyse enzymatique peut se conduire directement sur la biomasse prétraitée et/ou sur une biomasse prétraitée dont on a extrait les sucres solubles par exemple.

Dans certains cas, l'étape d'hydrolyse enzymatique de la cellulose est suivie d'une étape de fermentation, éthanolique par exemple, des sucres libérés et d'une étape de purification des produits de fermentation. Sous certaines configurations du schéma de procédé, les étapes d'hydrolyse enzymatique et de fermentation peuvent avoir lieu dans un même réacteur, dans une configuration de fermentation appelée SSF (Simultaneous Saccharification and Fermentation). Quand ces deux étapes du procédé sont séparées, on parle alors d'un schéma du type SHF (Separated Hydrolysis and Fermentation). Des exemples sont donnés par le document "Ethanol from lignocellulosics: A review of the economy", M. von Silvers et G. Zacchi, Bioresource Technology 56 (1996) 131-140.

Il est alors nécessaire d'extraire les sucres libres présents après l'étape de prétraitement en vue d'une utilisation dans une étape ultérieure sensible à la présence de solide, par exemple en cas de sensibilité du micro-organisme fermentaire mis en œuvre dans une étape ultérieure de fermentation, ou en cas de recyclage du micro-organisme autour de cette étape de fermentation ou pour faciliter l'opération de purification et/ou séparation des sucres ou des produits de fermentation que l'on pourrait obtenir à partir de ces sucres. Dans cette optique, la fraction liquide et la fraction solide sont séparées. La fraction solide se trouve sous la forme d'un gâteau, dans lequel une partie de la fraction liquide reste piégée de par les limitations des outils de séparations solide/liquide et les caractéristiques de la biomasse lignocellulosique.

On voit donc qu'à différentes étapes des procédés de traitement de biomasse, on a affaire à des produits sous forme de mélanges solide/liquide, et qu'il peut y a voir intérêt à extraire au moins une partie de la phase liquide de la phase solide en vue de la valoriser et d'optimiser le procédé global, et éventuellement réaliser le lavage de la phase solide pour maximiser la récupération des sucres solubles.

Le brevet WO 2014/135755 propose un procédé de traitement de biomasse avec la succession d'étapes suivantes : a- on effectue une étape de prétraitement par mise en contact et chauffage de la charge de biomasse avec de l'eau et un composé acide ou basique, de manière à obtenir un substrat prétraité, b- on met en contact le substrat prétraité avec des enzymes cellulases et avec un flux liquide enrichi en produits de fermentation obtenu à l'étape e) de manière à obtenir un hydrolysat comportant un résidu solide et une phase liquide contenant des sucres, c- on effectue une fermentation alcoolique de l'hydrolysat au moyen d'un microorganisme alcooligène de manière à produire un vin de fermentation comportant une matière solide et une phase liquide contenant des produits de fermentation, d- on extrait au moins une partie de la matière solide contenue dans le vin de fermentation de manière à obtenir un flux enrichi en matière solide et un vin de fermentation appauvri en matière solide, e- on lave le flux enrichi en matière solide avec un flux liquide de manière à obtenir ledit flux liquide enrichi en produits de fermentation, le flux liquide enrichi en produits de fermentation étant recyclé à l'étape b), f- on effectue une étape de séparation du vin de fermentation appauvri en matière solide de manière à obtenir au moins un flux purifié comportant un alcool ou un solvant et au moins un flux de vinasse.

Ce brevet propose ainsi d'effectuer la séparation de la lignine et d'autres éventuels solides inertes après l'étape de fermentation. La matière solide composée majoritairement de lignine est ensuite soumise à un lavage à l'eau pour récupérer les produits de fermentation piégés, en particulier les alcools et les solvants. Le liquide résultant du lavage est ensuite recyclé dans l'unité d'hydrolyse enzymatique, qui peut être la même unité que l'unité de fermentation ou qui peut être distincte de l'unité de fermentation afin de ne pas apporter de la dilution aux flux existants.

Le brevet EP- 2 774 992 propose d'effectuer, dans un procédé comportant une étape de prétraitement, une étape d'hydrolyse enzymatique puis une étape de fermentation, d'extraire au moins une partie du résidu solide contenu dans l'hydrolysat de manière à obtenir un flux de résidu solide comportant de la lignine et un hydrolysat appauvri en résidu solide, puis le flux de résidu solide est lavé avec un flux liquide comme de l'eau de façon à récupérer une phase liquide enrichie en sucres, qui peut être recyclée à l'étape d'hydrolyse enzymatique pour pouvoir valoriser les sucres sans apporter de dilution des flux dans le procédé.

Le brevet EP-3 587 583 décrit un procédé visant à opérer une extraction des sucres libres en deux étapes sur une biomasse prétraitée obtenue par un prétraitement de type explosion vapeur : une première étape dite de « repulpage » qui consiste en la mise en contact d'un marc sec et d'un fluide de mélange par un mélangeur en continu, puis une deuxième étape d'extraction/lavage mise en œuvre par un filtre en continu, utilisant un fluide de lavage pour obtenir une phase solide enrichie en solide et appauvrie en sucres libres et une pluralité de phases liquides enrichies en sucres, avec un recyclage au moins partiel d'une phase liquide extraite du filtre en entrée du mélangeur en tant que fluide de mélange. Un recyclage partiel d'une autre phase liquide extraite du filtre est réalisé pour l'utiliser en tant que fluide de lavage.

Ces différents traitements de biomasse impliquent donc une extraction des sucres libres ou de leur produits de fermentation d'une biomasse éventuellement déjà traitée / convertie au moins en partie pour en extraire des sucres libres dans une phase liquide valorisable.

Pour augmenter l'efficacité de ce lavage, qu'on peut aussi exprimer comme le rendement d'extraction en sucres libres, on peut à iso quantité d'eau de mélange et d'eau de lavage accroître le nombre d'étages théoriques de l'opération, adopter un fonctionnement en contre-courant ou à courants croisés plutôt qu'en co-courants. Par ailleurs, une augmentation de la quantité d'eau de lavage (et/ou de mélange) à iso configuration va se traduire par une amélioration du rendement d'extraction, mais aussi par une dilution accrue des sucres libres dans le jus extrait. Or, pour récupérer du jus les sucres valorisables, on procède usuellement à des opérations de concentration des jus, notamment par chauffage/évaporation, et plus le jus sera dilué, plus la consommation énergétique sera importante pour le concentrer

L'invention a alors pour but d'améliorer la façon de procéder à une opération d'extraction des sucres libres de biomasse lignocellulosique après un ou plusieurs traitements, notamment en réduisant la consommation énergétique/les coûts de leur traitement/valorisation ultérieur(e).

### Résumé de l'invention

L'invention a tout d'abord pour objet un procédé de traitement d'une biomasse lignocellulosique préalablement prétraitée, ledit procédé comprenant l'extraction des sucres libres de ladite biomasse prétraitée par une solution d'extraction alcoolique ou hydro-alcoolique, de façon à obtenir une phase liquide enrichie en sucres libres, appelée jus, et une phase solide appauvrie en sucres libres, appelée marc.

Il a été démontré, avec l'invention, que procéder à l'extraction des sucres libres de la biomasse lignocellulosique prétraitée avec une solution alcoolique ou hydro-alcoolique permettrait de réduire les coûts associés à la concentration des jus de sucres extraits, et cela sans dégrader l'efficacité d'extraction des sucres. Or c'est un résultat tout-à -fait surprenant, dans la mesure où les alcools sont plutôt de mauvais solvants des sucres (solubilité des sucres inférieure à 1% poids dans le cas de l'éthanol, qui est d'ailleurs plutôt connu pour faciliter la cristallisation des sucres, c'est-à-dire tout le contraire de ce qui est recherché dans le cadre de la présente invention).

De préférence, la solution alcoolique ou hydroalcoolique utilisée pour l'extraction selon l'invention ne contient aucun autre composé que de l'alcool (ou un mélange d'alcools) et éventuellement de l'eau. Elle ne contient, notamment, ni acide ni base ni agent oxydant.

L'extraction des sucres libres selon l'invention s'est révélée également avantageuse sur au moins deux autres aspects :
D'une part, il s'est avéré que l'extraction selon l'invention des sucres libres présents dans le mélange solide/liquide obtenu après prétraitement de la biomasse lignocellulosique permettait d'améliorer les étapes de purification des sucres avant leur concentration. La biomasse lignocellulosique prétraitée contient généralement, en effet, une quantité non négligeable de sels minéraux, et tout particulièrement des anions sulfates SO₄ ²⁻ quand la biomasse a été prétraitée par de l'acide sulfurique. Un moyen efficace pour les éliminer connu de l'homme du métier est de réaliser une étape de chaulage couramment pratiquée sur les jus de sucres obtenus à partir de biomasse de première génération 1G, c'est-à-dire l'ajout d'un lait de chaux contenant de l'hydroxyde de calcium. Ce traitement permet de faire précipiter, entre autres, les anions sulfates sous forme de sulfate de calcium. Il s'est avéré que réaliser un chaulage sur un jus de sucres obtenu selon l'invention avec une extraction des sucres libres par une solution alcoolique ou hydro-alcoolique, permettait d'éliminer davantage de sulfates, réduisant ainsi le coût des opérations ultérieures de traitement.

D'autre part, il se trouve que la biomasse ainsi traitée retient une (faible) portion de la solution de lavage, donc une (faible) portion d'alcool selon l'invention. Et les jus sucrés extraits contiennent aussi une certaine proportion d'alcool. Dans l'un et l'autre cas, cette présence d'alcool est favorable car elle tend à limiter la contamination microbienne du marc et du jus sucré. La contamination microbienne est en effet un problème fréquemment rencontré industriellement, et la présence d'alcool peut éviter d'avoir recours à des traitements antimicrobiens, notamment par ajout d'antibiotiques ou autres composés.

Avantageusement, la biomasse est préalablement prétraitée par imprégnation avec une solution aqueuse acide, basique ou oxydante, puis cuisson ou explosion à la vapeur. C'est un prétraitement connu, dont un exemple est décrit dans le brevet EP 3728408.

De préférence, l'extraction des sucres est réalisée sur une biomasse prétraitée qui présente un taux de matière sèche insoluble MSI d'au moins 20%, notamment d'au moins 25% ou d'au moins 30%, et de préférence d'au plus 70 ou 65%, notamment compris entre 25% et 65%.

Le taux de matière sèche insoluble MSI peut être déterminé de la façon suivante : on filtre sous vide l'échantillon en réalisant des rinçages successifs à l'eau osmosée avec un ratio massique compris entre 40 et 100 puis on sèche l'échantillon à 105°C jusqu'à obtenir une masse constante. La masse perdue pendant le lavage, la filtration et le séchage correspond à la masse d'eau (de liquide) présente initialement dans l'échantillon ainsi qu'aux matières solubles contenues dans la fraction liquide, la masse restante correspond aux matières sèches insolubles MSI.

De préférence, l'extraction des sucres est réalisée sur une biomasse prétraitée qui présente un taux de matière sèche MS d'au moins 25%, notamment d'au moins 30% ou d'au moins 35%, et de préférence d'au plus 70 ou 65%, notamment compris entre 30% et 65%.

Le taux de matière sèche, MS, est déterminé selon la norme ASTM E1756-01 qui consiste à mesurer la perte de masse de la fraction liquide par séchage à 105°C jusqu'à obtenir une masse constante de résidu. La masse perdue pendant le séchage correspond à la masse d'eau (de liquide) présente initialement dans l'échantillon, et la masse restante correspond aux matières solides et solubles contenues dans la fraction liquide.

Selon une variante, on peut prévoir de faire précéder l'extraction des sucres par une étape d'ajustement du taux de matière sèche MS de la biomasse prétraitée, notamment par une opération de séparation solide/liquide préalable, de préférence du type filtration ou centrifugation.

De préférence, l'extraction des sucres de la biomasse prétraitée selon l'invention comporte un contactage de ladite biomasse prétraitée avec la solution d'extraction alcoolique ou hydro-alcoolique, notamment une mise en suspension dans ladite solution, puis une séparation solide/liquide.

Optionnellement, l'extraction des sucres selon l'invention peut comporter, après la séparation solide/liquide, un lavage de la phase solide obtenue par ladite séparation avec une solution de lavage alcoolique ou hydro-alcoolique. Le lavage est alors de préférence opéré en continu, en co-courant ou contre-courant.

La solution d'extraction et la solution de lavage, quand un lavage est prévu, peuvent être de même composition, avec le ou les mêmes alcools, ou avoir un ou des alcools différents. Dans le cas des solutions hydro-alcooliques, leurs teneurs en alcool par rapport à l'eau peuvent aussi être identiques ou différentes. Ainsi, dans le cas où l'extraction des sucres et le lavage de la phase solide obtenue à l'issue de la séparation solide/liquide est étagé, différentes proportions d'alcool peuvent être utilisées. Réaliser un lavage final de la phase solide par une solution aqueuse ne contenant pas d'alcool est également possible.

Dans le cadre de la présente invention, quand une séparation solide/liquide est réalisée, la « phase solide » séparée est à comprendre comme contenant la phase solide elle-même et la phase liquide piégée dans ladite phase solide.

Le contactage de la biomasse prétraitée avec la solution alcoolique ou hydro-alcoolique peut être opéré par un mélangeur en continu (comme décrit dans le brevet EP-3 587 583 précité) ou en discontinu, en une ou plusieurs étapes/étages.

La séparation solide/liquide peut être opérée en discontinu ou en continu, notamment par un filtre-presse ou un filtre à bande (comme décrit dans le brevet EP-3 587 583 précité).Avantageusement, la solution d'extraction et/ou de la solution de lavage quand un lavage est prévu, comprend au moins un alcool choisi parmi : méthanol, éthanol, isopropanol, n-butanol, isobutanol. L'alcool préféré est l'éthanol.

De préférence, la solution d'extraction et/ou de la solution de lavage quand un lavage est prévu, est une solution hydro-alcoolique contenant au moins 5% poids d'alcool, notamment au moins 10% poids, de préférence entre 5% poids et 96% poids, ou entre 10% poids et 85% poids, ou entre 15% poids et 50 ou 70% poids d'alcool. Avec cette proportion d'alcool dans l'eau, on a une solution qui procure l'effet voulu sans atteindre un niveau de consommation d'alcool trop élevée qui ne serait pas économiquement intéressante.

De manière très préférée, la teneur en alcool est comprise entre 15 et 50% poids, de manière à augmenter le point éclair de la solution hydro-alcoolique pour s'affranchir de certaines contraintes industrielles, et à limiter les pertes en alcool par évaporation. A noter aussi qu'une solution hydro-alcoolique est plus facile à manipuler et à stocker dans une installation industrielle que de l'alcool pur.

De fait, le choix de la proportion d'alcool dans la solution d'extraction (et/ou de lavage quand lavage il y a) dépend notamment de la teneur en eau dans la biomasse prétraitée avant extraction, qui peut largement varier d'un type de biomasse à un autre. (Pour stabiliser/modifier cette teneur, l'invention peut, comme déjà indiqué plus haut, ajuster le taux de MS de la biomasse prétraitée avant extraction).

Une solution consiste à ajuster la teneur en alcool de la solution d'extraction hydro-alcoolique, et également le rapport du débit massique de ladite solution sur le débit massique de biomasse pour que la teneur en alcool dans le jus obtenu soit d'au plus 70% poids hors soluté, de préférence d'au plus 50% poids hors soluté, et de préférence d'au moins 10% poids hors soluté.

On peut ainsi régler le taux d'alcool (alcool/ eau + alcool) dans la solution d'extraction en fonction du taux d'alcool voulu (alcool/ eau + alcool) -hors sucres- dans le jus obtenu.

On peut sélectionner des paramètres de fonctionnement de l'étape de contactage entre la biomasse prétraitée et la solution d'extraction pour que, in fine, on ait le taux de MSI voulu pour faire la séparation puis l'éventuel lavage, notamment en fonction du type d'équipements choisis pour faire la séparation, et de l'efficacité-cible de séparation visée.

De préférence, l'extraction selon l'invention est réalisée à température modérée, inférieure ou égale à100°C, notamment inférieure ou égale à 80°C, notamment inférieure à 80°C, et par exemple d'au moins 10°C, notamment entre 15 et 75°C ou entre 20 et 60°C. En fait elle peut se faire à température ambiante (donc par exemple entre 10 et 20-25°C).

De préférence, le contactage de la biomasse prétraitée (et/ou le lavage quand il est prévu) est réalisé à température modérée, et même à température ambiante, notamment à une température inférieure ou égale à 100°C, notamment comprise entre 10 et 80°C, de préférence entre 20 et 60°C. La température en question s'entend comme la température du mélange dans l'étape de contactage de la biomasse avec une solution alcoolique ou hydro-alcoolique. De manière préférée dans cette configuration, la régulation de la température du mélange se fait en régulant principalement la température de la solution d'extraction alcoolique ou hydro-alcoolique. (La température s'entend comme la température de la solution de lavage alcoolique ou hydro-alcoolique dans l'étape de lavage si elle est prévue).

On peut traiter le jus obtenu de différentes manière pour valoriser les sucres extraits:
- on peut convertir les sucres libres du jus en furfural, notamment par déshydratation par catalyse acide,
- ou on peut convertir les sucres libres du jus en xylitol et/ou sorbitol, notamment par hydrogénation par catalyse métallique,
- ou encore on peut les convertir en alcool, notamment par fermentation alcoolique.

On peut traiter le jus obtenu pour l'appauvrir en sels minéraux, notamment par chaulage et/ou par mise en contact avec un ou des substrat(s) actifs du type résine(s) échangeuse(s) d'ions et/ou par électrodialyse.

Eventuellement, le jus peut être traité avec d'autres substrats, de type charbon actif par exemple, pour l'appauvrir en autres composés, le charbon actif étant ainsi apte à capter des composés colorés contenus dans le jus.

Avantageusement, les jus de sucres libres peuvent donc être traités par chaulage. Une opération de chaulage consiste à ajouter au jus de sucres libres extraits une solution de lait de chaux vive, c'est-à-dire une solution aqueuse d'hydroxyde de calcium Ca(OH)₂ saturée. La concentration de la solution de lait de chaux ajoutée est comprise entre 5 et 40% poids, notamment entre 10 et 20% poids. Le mélange du lait de chaux et du jus de sucres est réalisé à une température comprise entre 20 et 80°C. Le pH du mélange obtenu est compris entre 8 et 11,5, de préférence entre 9 et 10,5. Les anions hydroxyles HO⁻ permettent la neutralisation du pH acide induits par les protons H+, et induisent la formation d'eau. Les cations calcium Ca2⁺ induisent une précipitation partielle des ions sulfates SO₄²⁻, sous la forme de sulfate de calcium. Le précipité formé peut être éliminé par filtration, décantation ou centrifugation. L'opération de chaulage peut aussi permettre d'éliminer divers contaminants, principalement inorganiques, mais aussi organiques.

Le chaulage sur un jus de sucres produit selon l'invention, c'est-à-dire une solution hydro-alcoolique de sucres, est nettement plus performant qu'un chaulage sur une solution aqueuse de sucres. Néanmoins, il ne permet pas toujours de réduire la concentration des sels à de très faibles teneurs. Dans ce cas, on peut compléter le chaulage ou le remplacer par une opération visant à supprimer davantage de/totalement les sels minéraux, notamment dans le cas où l'on prévoit ensuite de concentrer le jus de sucres.

Pour une suppression très efficace des sels, on peut utiliser une résine échangeuse d'ions : on fait faire passer le jus (éventuellement préalablement chaulé) au travers de résines échangeuses d'anions sous forme hydroxyde, puis au travers de résines échangeuses de cations sous forme protonée. Les anions et les cations présents dans la solution aqueuse de sucres libres sont retenus sur les résines échangeuses d'anions et de cations respectivement, et libèrent des anions hydroxyde et des protons respectivement. Ces derniers réagissent ensemble de manière totale pour former de l'eau. Les résines saturées peuvent ensuite être régénérées.

On peut aussi utiliser la technique d'électrodialyse, qui fonctionne sur le même principe que les résines échangeuses d'ions, mais l'échange des ions se fait au travers de membranes sélectives, sous l'effet d'un courant électrique produisant les anions hydroxydes et les protons nécessaires.

Lorsqu'une résine échangeuse d'ions et/ou l'électrodialyse est utilisée sur une solution de jus sucrée produite par la présente invention, cela peut être réalisé sur la solution produite par l'invention, sur la solution produite par l'invention qui a subi un chaulage préalable, sur la solution produite par l'invention de laquelle on a retiré le solvant (éthanol, ...) par évaporation et/ou distillation en complément ou non du chaulage.

On peut aussi concentrer en sucres le jus obtenu, notamment par évaporation, éventuellement après et/ou avant un traitement d'appauvrissement en sels minéraux dudit jus. L'étape de concentration du jus de sucres produit par la présente invention peut être réalisée selon toute technique connue de l'homme du métier, par exemple par évaporation multi-effet ou avec compression mécanique de la vapeur.

La concentration peut être réalisée avec récupération de l'alcool et/ou de l'eau pour constituer tout ou partie de l'alcool et/ou de l'eau de la solution d'extraction alcoolique ou hydro-alcoolique utilisée et/ou pour la solution de lavage quand un lavage est prévu : La concentration du jus peut être réalisée par évaporation et on peut alors récupérer de l'alcool et/ou de l'eau pour constituer tout ou partie de l'alcool ou de la solution hydro-alcoolique nécessaire à la réalisation de l'invention (extraction et/ou lavage éventuel) : on peut ainsi recycler, au moins en partie, le ou les constituants de la solution alcoolique ou hydro-alcoolique. Les condensats obtenus après concentration du jus de sucres selon l'invention, principalement constitués d'alcool et d'eau peuvent être distillés dans une colonne de distillation pour séparer tout ou une partie de l'alcool de l'eau.

Selon un mode de réalisation, on peut traiter le jus obtenu pour l'appauvrir en sels minéraux (à des concentrations élevées à chaud), puis le concentrer, puis enfin opérer une cristallisation (à froid) sur ce jus appauvri en sels minéraux et concentré en sucres.

L'invention a également pour objet un procédé tel que décrit plus haut et qui comprend une hydrolyse enzymatique du marc obtenu après extraction, qui est suivie de, ou est concomitante à, une fermentation alcoolique, puis une distillation de la fraction liquide obtenue après fermentation. Ainsi, une partie de l'alcool et/ou tout ou partie de l'eau récupérée lors de la distillation peuvent être utilisée(s) pour constituer tout ou partie de la solution d'extraction alcoolique ou hydro-alcoolique (et/ou de la solution de lavage quand un lavage est prévu).

L'invention a également pour objet un procédé de traitement d'une biomasse lignocellulosique qui comprend les étapes suivantes successives :
- imprégnation de la biomasse par une solution aqueuse acide, basique ou oxydante, pour obtenir une biomasse imprégnée,
- prétraitement de la biomasse imprégnée par cuisson ou explosion à la vapeur, pour obtenir une biomasse prétraitée,
- hydrolyse enzymatique de la biomasse prétraitée, pour obtenir une biomasse hydrolysée,
- éventuellement fermentation alcoolique de la biomasse hydrolysée,
et tel qu'on opère par une solution d'extraction alcoolique ou hydro-alcoolique une extraction des sucres libres sur tout ou partie de la biomasse prétraitée avant son hydrolyse enzymatique.

Selon l'invention, on peut ainsi prévoir d'opérer l'extraction des sucres sur toute la biomasse prétraitée, ou seulement sur une partie de celle-ci.

Dans ce dernier cas de figure, on prévoit de préférence que l'ensemble de la partie de la biomasse prétraitée ayant subi l'extraction des sucres et de la partie qui ne l'a pas subi soit traitée par hydrolyse enzymatique.

L'extraction des sucres libres selon l'invention peut comporter un recyclage au moins partiel d'une phase liquide extraite du dispositif du type filtre opérant la séparation solide/liquide vers l'entrée du dispositif du type mélangeur opérant le contactage. Ce recyclage permet de diminuer la consommation en solution alcoolique ou hydro-alcoolique, et s'est avéré, de façon surprenante, permettre à la fois un mélange plus efficace au niveau du mélangeur et une extraction de phase(s) liquide(s) au niveau du filtre plus concentrée(s) en sucres.

Ces divers recyclages possibles permettent de diminuer la consommation en alcool et en eau du procédé de lavage, et/ou de réduire les consommations énergétiques. Ils peuvent être conduits individuellement ou en complémentarité. L'intégration thermique entre les diverses étapes optionnelles est possible, et même recommandée.

En termes de conditions de pression utilisée lors de l'extraction : la pression d'opération dépend de l'étape considérée (contactage, séparation solide/liquide et éventuel lavage), de la technologie retenue et du point de mesure. Dans de nombreuses technologies, la pression est l'élément moteur permettant de faire circuler la phase liquide permettant l'extraction et/ou le lavage. De manière générale, les technologies ont toutes un fluide à pression atmosphérique et un différentiel de pression entre 0,2 et 25 bars entre les fluides entrants et sortants, et de manière encore plus préférée, entre 0,4 et 20 bars. Ce différentiel de pression peut être négatif lors de la séparation solide/liquide. Ce différentiel de pression peut aussi être obtenu par l'application d'une pression externe sur le mélange solide/liquide comme dans le cas d'une presse à bande, ou lors de l'utilisation d'une presse en option sur un filtre à bande.

Par exemple, dans le cas de l'utilisation d'un filtre-presse pour la séparation solide/liquide, le fluide extrait du filtre est à pression atmosphérique et le fluide entrant est à une pression plus élevée. Cette pression est variable en fonction de la phase d'opération. Lors de la filtration, la pression du fluide à filtrer augmente entre la pression atmosphérique et une pression maximale dépendante de l'équipement, généralement comprise entre 5 et 15 bars. Lors de l'étape de lavage éventuel, la pression du fluide lavage est constante, et généralement supérieure à la pression en fin de filtration et généralement comprise entre 6 et 20 bars.

Par exemple encore, lors de l'utilisation d'un filtre à bande pour la séparation solide/liquide, le mélange est déposé sur le filtre et une dépression est appliquée sous le filtre : le liquide est ainsi tiré du « gâteau » déposé sur le filtre et passe à travers le filtre avant d'être collecté dans des bacs. La dépression appliquée est généralement comprise entre 0,2 et 2 bars, préférentiellement entre 0,4 et 1 bar. L'opération est séquentielle, la bande se déplace selon une séquence de temps définie, lors du déplacement de la bande, il n'y a pas de dépression appliquée. Eventuellement, une presse peut être ajoutée afin d'augmentation la pression appliquée sur le mélange déposé sur la bande et augmenter l'extraction de liquide.

### Liste des figures

Figure 1

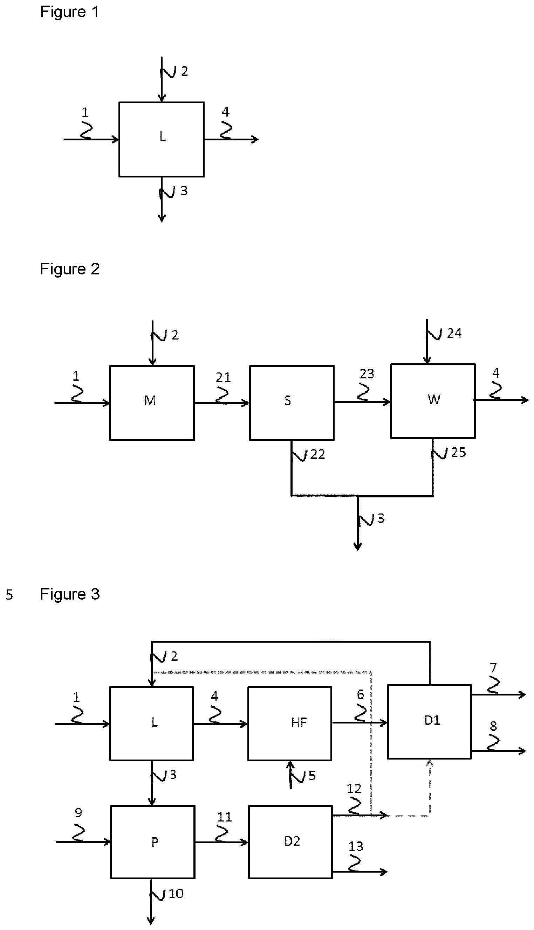

   La figure 1 représente un schéma-bloc d'une opération d'extraction des sucres selon un premier mode de réalisation de l'invention.
Figure 2
   La figure 2 représente un schéma-bloc d'une opération d'extraction des sucres selon un deuxième mode de réalisation de l'invention.
Figure 3
   La figure 3 représente un schéma-bloc d'une opération d'extraction des sucres selon un troisième mode de réalisation de l'invention.

### Description des modes de réalisation

L'invention sera ci-après détaillée à l'aide d'exemples non limitatifs illustrés par les figures 1, 2 et 3. Les mêmes références correspondent aux mêmes composants / fluides / produits sur l'ensemble des figures. Les figures sont extrêmement schématiques.

Il a été découvert, avec la présente invention, qu'il était possible et très avantageux de réaliser une extraction des sucres libres de la biomasse prétraitée avec un alcool ou un mélange hydro-alcoolique. De manière préférée, l'alcool utilisé est de l'éthanol, ce qui parait au premier abord un choix allant à rencontre du but recherché, qui est d'extraire les sucres libres de la biomasse, car l'éthanol est plutôt un mauvais solvant des sucres (solubilité des sucres inférieurs à 1%pds). L'éthanol est connu pour être un « anti-solvant » des sucres, utilisé pour faciliter leur cristallisation.

Avantageusement, l'alcool ou le mélange hydro-alcoolique utilisé pour l'extraction des sucres libres peut être produit par une étape de fermentation de tout ou une partie des sucres libres récupérés à l'étape de lavage de la biomasse prétraitée.

Avantageusement, l'alcool ou le mélange hydro-alcoolique utilisé pour l'extraction des sucres libres de la biomasse prétraitée peut être produit par hydrolyse enzymatique et fermentation de la biomasse prétraitée extraite appelée marc lavé..

On peut ainsi intégrer le procédé d'extraction des sucres libres au procédé complet de conversion de biomasse dans une installation industrielle de production d'éthanol, ou de production d'éthanol et de sucres.

L'utilisation d'un alcool ou d'un mélange hydro-alcoolique pour extraire les sucres libres de la biomasse permet d'obtenir des rendements d'extraction des sucres libres au moins équivalents à l'utilisation de l'eau comme solvant, toutes choses égales par ailleurs (débits, performance et mode de fonctionnement de l'outil de séparation liquide-solide).

Par contre, l'utilisation d'un alcool ou d'un mélange hydro-alcoolique pour extraire les sucres libres de la biomasse permet d'obtenir des avantages supplémentaires par rapport à une solution de lavage purement aqueuse : réduction de la consommation énergétique associée à la concentration des sucres libres extraits dans le jus ; réduction des coûts associés à l'élimination des sels ; limitation des risques de contaminations microbiennes du marc lavé et de la solution de sucres libres extraits de la biomasse.

De manière préférée, le rapport de masse de solution alcoolique ou hydro-alcoolique sur la masse de biomasse (sur une base de matière sèche insoluble) mises en œuvre dans la présente invention est compris entre 0,1 et 100. De manière préférée, le rapport de masse de solution alcoolique ou hydro-alcoolique sur la masse de biomasse est compris entre 1 et 10. Le rapport de masse peut se faire sur le rapport des débits massiques lorsque l'opération est à alimentation continue, ou sur le rapport des masses engagées sur un cycle lorsque l'opération est une opération batch.

La biomasse produite à l'étape de prétraitement contient une quantité d'eau qui peut être variable, selon le type et les conditions du prétraitement. L'extraction des sucres libres peut comprendre une étape de mise en contact avec une solution alcoolique ou hydro-alcoolique. L'extraction des sucres libres comprend au moins une étape de séparation liquide-solide. L'extraction des sucres libres peut comprendre une étape de lavage du solide obtenu après séparation du mélange solide/liquide par une solution alcoolique ou hydro-alcoolique.

De manière préférée, on ajuste la teneur en alcool dans la solution hydro-alcoolique et le rapport de masse de solution hydro-alcoolique sur la masse de biomasse, pour que la teneur en alcool dans le jus de sucres libres extraits soit inférieure à 70% poids (hors soluté), de préférence inférieure à 50% poids (hors soluté).

De manière préférée, on ajuste la teneur en alcool dans la solution hydro-alcoolique et le rapport de masse de solution hydro-alcoolique sur la masse de biomasse prétraitée, pour que la teneur en alcool dans le jus de sucres libres extraits soit supérieure à 10% poids (hors soluté).

L'opération de lavage du solide obtenu après éventuellement mise en contact de la biomasse prétraitée avec une solution alcoolique ou hydro-alcoolique et après séparation liquide/solide est réalisée à une pression fonction de la technologie retenue, de préférence avec un différentiel de pression entre l'entrée de la solution de lavage et la sortie du jus sucré compris entre 0,2 et 25 bars, et préférentiellement entre 0,4 et 20 bars. De manière préférée, au choix au moins une entrée ou au moins une sortie de liquide est à pression atmosphérique.

L'opération d'extraction des sucres libres de la biomasse peut être réalisée par toutes techniques d'extraction liquide-solide connues de l'homme du métier. Cette extraction liquide-solide peut être réalisée en un ou plusieurs étages de contacts. Si plusieurs étages de contact sont mis en œuvre, la solution ou les solutions hydro-alcooliques peu(ven)t être mise en œuvre en courants croisés, à co-courant ou à contre-courant de la biomasse.

L'opération d'extraction des sucres libres peut être discontinue ou continue. Dans le cas d'une opération à courants croisés, les solutions hydro-alcooliques successivement mises en contact avec la biomasse peuvent avoir des teneurs en alcools différentes.

De manière non limitative, l'extraction des sucres libres peut être réalisée par une dispersion préalable de la biomasse dans une solution hydro-alcoolique, puis une séparation liquide-solide à l'aide d'un outillage tel qu'un filtre presse ou un filtre à bande.

Avantageusement, la solution hydro-alcoolique est produite sur site, par fermentation d'une fraction des sucres de la biomasse lignocellulosique en alcool.

Dans un mode de réalisation, la biomasse prétraitée et débarrassée de ses sucres libres par le procédé selon l'invention, appelé marc lavé, peut être ensuite traité dans une étape d'hydrolyse et fermentation dans le but de produire de l'éthanol. De manière non limitative, le moût obtenu en fin de fermentation, contenant approximativement entre 4% poids et 10% poids d'éthanol peut être filtré pour le débarrasser du solide résiduel, puis être utilisé tel que pour réaliser l'invention. De manière non limitative, le moût obtenu en fin de fermentation, contenant approximativement entre 4% poids et 10% poids d'éthanol peut être filtré pour le débarrasser de la biomasse et de la lignine ou de tout autre solide résiduel, puis distillé selon toute technique connue de l'homme du métier, dans une colonne de distillation ou colonne à bière. Par un soutirage latéral de la colonne, on peut s'approvisionner en un mélange eau-éthanol ayant une teneur en alcool comprise entre 10% poids et 96% poids, et qui peut être utilisé pour reconstituer une solution hydro-alcoolique nécessaire à la réalisation de l'invention.

Dans un mode de réalisation, une fraction des sucres libres présents dans le jus obtenu par réalisation de l'invention subit un traitement (ajustement du pH notamment) de manière à pouvoir être traitée par fermentation alcoolique. A titre d'illustration, cette fermentation peut être une fermentation éthanolique (en présence de levure) ou une fermentation en un mélange acétone / butanol / éthanol ou encore une fermentation en un mélange isopropanol / butanol / éthanol. Le moût de fermentation peut être distillé pour récupérer des solutions d'eau et d'alcool(s) ou de mélanges eau + alcool, qui peuvent être utilisées pour réaliser l'invention.

L'invention peut s'appliquer à tout type de biomasse lignocellulosique prétraitée, par exemple provenant de biomasse de type résidus forestiers, taillis à croissance rapide, Miscanthus, résidus agricoles, comme des résidus céréaliers de type paille (de blé) ou résidus de maïs. Avant prétraitement, la biomasse pourra, de façon connue, être conditionnée avec notamment un traitement mécanique (broyage) de façon à obtenir des particules de biomasse ayant de préférence une taille d'au plus 300 mm. De façon générale, la biomasse conditionnée présente une taille de particule (la plus grande taille) d'au plus 300 mm, le plus souvent d'au moins 1 mm, et souvent comprise entre 2 et 200 mm.

Un exemple non limitatif de mise en œuvre de l'invention consiste à réaliser :
a- une étape de prétraitement explosif, comme par exemple un prétraitement par explosion vapeur de la biomasse lignocellulosique après son imprégnation par une solution acide ;
b- une étape d'extraction des sucres libres de la biomasse prétraitée obtenue à l'étape a- à l'aide d'une solution hydro-alcoolique avec une mise en contact de la biomasse prétraitée et de la solution hydro-alcoolique, une séparation solide/liquide et éventuellement un lavage du solide par la solution hydro-alcoolique, afin de récupérer dans un jus les sucres libérés par la biomasse lors de son prétraitement et un marc (lavé) ;
c- une étape de valorisation des sucres du jus récupéré à l'étape b-
d- une éventuelle étape de valorisation du marc (lavé) obtenu à l'étape b-

Un exemple non limitatif de mise en œuvre de l'étape c) consiste à réaliser :
c1- une étape de chaulage
c2- une première étape de concentration
c3- une étape de déminéralisation par résine ou électrodialyse
c4- une seconde étape de concentration
c5- une étape de cristallisation

Un exemple non limitatif de mise en œuvre de l'étape d- consiste à réaliser :
d1- une étape de mise en solution du marc (lavé)
d2- une hydrolyse enzymatique de la fraction cellulosique du marc
d3- une étape de fermentation éthanolique des sucres libérés par l'étape d2-, éventuellement couplée à d2-
d4- une étape de distillation du moût de fermentation, permettant de produire de l'éthanol, de l'eau, et la solution hydro-alcoolique nécessaire à la réalisation de l'étape b-.

### Description des figures

En référence à la figure 1, une biomasse prétraitée 1 obtenu par prétraitement de biomasse lignocellulosique subit une opération d'extraction des sucres libres L par une solution hydro-alcoolique 2 de type eau-éthanol. Elle produit un marc lavé 4 et un jus 3 composé d'une solution hydro-alcoolique de sucres libres extraits.

La figure 2 représente une extraction des sucres selon l'invention qui comporte une opération de contactage de la biomasse prétraitée avec une solution alcoolique ou hydro-alcoolique, puis une séparation solide/liquide, et un lavage de la phase solide obtenue après séparation avec une solution de lavage alcoolique ou hydro-alcoolique. En référence à la figure 2, une biomasse prétraitée 1 obtenue par prétraitement de la biomasse lignocellulosique est mise en contact dans un mélangeur M avec une solution hydro-alcoolique 2 de type eau-éthanol. Le mélange solide/liquide 21 subit ensuite une séparation solide/liquide S. Elle produit une phase liquide 22 composée d'une solution hydro-alcoolique de sucres libres extraits et une phase solide 23 appauvrie en sucres libres.

La phase solide 23 subit une opération de lavage W avec une solution hydro-alcoolique 24 pour produire un marc lavé 4 plus appauvri en sucres libres que la phase solide 23, et un filtrat de lavage 25 composée d'une solution hydro-alcoolique de sucres libres. Cette solution 24 peut être de même composition que la solution d'extraction 2, ou être de composition différente.

La phase liquide 22 obtenue par séparation solide/liquide et le filtrat de lavage 25 peuvent être mélangés pour constituer le jus 3 de sucres libres dans une solution hydro-alcoolique.

La succession des étapes de contactage, séparation solide/liquide et lavage permet d'augmenter la récupération des sucres libres dans le jus.

A noter que l'invention peut prévoir des recyclages de fluides, notamment, par exemple, de la solution 25 pour compléter ou remplacer la solution d'extraction 2.

En référence à la figure 3, une biomasse obtenue par prétraitement de biomasse lignocellulosique 1 subit une opération d'extraction des sucres libres L par une solution hydro-alcoolique 2 de type éthanol + eau. Elle produit un marc lavé ? 4 et une solution hydro-éthanolique de sucres libres extraits : le jus 3. L'opération d'extraction des sucres libres L comprend un contactage de la biomasse prétraitée 1 avec le mélange 2 eau-éthanol, puis une séparation solide/liquide et optionnellement une étape de lavage (comme avec le flux 24 de la figure 2) de la phase solide obtenue par la séparation avec le mélange 2 eau-éthanol.

A noter que l'extraction L peut être réalisée en une étape comme à la figure 1 ou en plusieurs étapes comme à la figure 2.

Le marc lavé 4 subit ensuite une opération HF d'hydrolyse enzymatique et une opération de fermentation alcoolique, dissociées/successives ou simultanée, dans le but de produire une solution aqueuse d'éthanol 6. A cette fin, le marc lavé est mis en contact avec une ou plusieurs solutions aqueuses 5 contenant les enzymes et les levures nécessaires à cette transformation. La solution aqueuse d'éthanol 6 ainsi produite subit ensuite une opération de distillation D1 pour produire de l'éthanol 7 et des vinasses 8. Le flux d'éthanol 7, de façon connue, n'est pas nécessairement pur et peut encore contenir de l'eau.

Une solution hydro-alcoolique 2 est soutirée de la colonne à distiller D1 afin d'alimenter l'opération d'extraction L.

La solution hydro-éthanolique de sucres libres extraits (jus) 3 subit une ou plusieurs étapes optionnelles de purification, ici sous forme d'un chaulage (c'est-à-dire appauvris en certains sels minéraux du type sulfates). Le flux 9 représente le ou les agents de séparation (hydroxyde de calcium, résines,...) destinés à appauvrir le jus 3 de ses sels minéraux, notamment de type sulfates provenant de l'acide sulfurique utilisé pour prétraiter la biomasse. Ce flux 9 est mis en contact avec la solution hydro-éthanolique de sucres libres extraits 3. Le flux 10 représente le ou les agents de séparation qui ont capté les sels minéraux (sulfate de calcium, résines saturées,...), qui sont ensuite remplacés ou régénérés.

La solution hydro-éthanolique de sucres libres purifiés (jus appauvri en sels) 11 subit une opération de concentration par évaporation D2 dans le but de produire une solution aqueuse de sucres libres purifiés concentrés 13 et une solution hydro-alcoolique 12. Cette solution hydro-alcoolique 12 peut avantageusement être directement recyclée à l'étape d'extraction des sucres libres du marc L (ligne en pointillés sur la figure), ou indirectement recyclée en alimentant la colonne à distiller D1.

### Exemples

Une biomasse lignocellulosique, paille de blé, contenant 30% poids d'hémicellulose dont la teneur en matière sèche est de 90% poids et la teneur en matière sèche insoluble est de 80% poids subit un prétraitement qui consiste en :
- une imprégnation par une solution aqueuse d'acide sulfurique : l'imprégnation est effectuée en présence de liqueur acide chauffée à 80°C et un temps de séjour de 2 minutes. La concentration en acide dans la liqueur est de 2,5% poids (exprimé en % poids d'H₂SO₄). Le taux de matière sèche MS de la biomasse imprégnée est de 25% poids.
- l'introduction de cette biomasse imprégnée au sein d'un réacteur sous pression chauffé à 180°C avec injection de vapeur, pendant un temps de séjour de 5 min, permettant un rendement de conversion de l'hémicellulose vers le xylose monomérique de 70% poids ;
- une détente à pression atmosphérique libérant de la vapeur et une biomasse prétraitée ayant un taux de matière sèche de 50% poids et un taux de matière sèche insoluble de 40% poids.

La biomasse prétraitée ainsi produite est utilisée pour produire les exemples 1 et 2 (non conformes), et 3 et 4 (conformes à l'invention).

L'extraction des sucres libres présents dans la biomasse prétraitée, selon l'invention et pous les quatre exemples, se fait en deux temps.

1/ La biomasse prétraitée est d'abord repulpée, à température ambiante à un taux de matière sèche MS de 20% poids et un taux de matière sèche insoluble MSI de 16% poids, avec une solution aqueuse de mélange ayant des teneurs en éthanol comprises entre 0 et 70% poids, à raison de 1,3 g de solution de mélange par gramme de biomasse prétraitée. On comprend par « repulpage » l'opération consistant à mettre en contact la biomasse prétraitée avec la solution hydro alcoolique par mise en suspension de la biomasse dans la solution.

2/ La biomasse repulpée alimente un filtre-presse pour opérer une séparation solide/liquide avec l'obtention du jus de sucres et d'un marc lavé: La biomasse repulpée est d'abord filtrée sous pression à une température de 50°C, puis la phase solide retenue par la toile filtrante dans les chambres de filtration du filtre-presse est compactée en injectant de l'air pour presser. Le taux de matière sèche insoluble dans le marc lavé après séparation liquide-solide est d'environ 45% poids pour les différents exemples. La teneur en sucres dans les différents jus est déterminée par HPLC (Chromatographie phase liquide haute performance), et permet de déterminer un rendement d'extraction des sucres défini comme étant le ratio entre la quantité de sucres extraits dans le jus et la quantité de sucres présents dans la biomasse prétraitée de l'ordre de 75% pour tous les exemples. La teneur en sucres (glucose et xylose notamment) dans le jus obtenu est de 56 g/kg. La teneur en H₂SO₄ dans le jus obtenu est de l'ordre de 1,1% poids déterminée par chromatographie ionique. Optionnellement la phase solide retenue par la toile filtrante dans les chambres de filtration du filtre-presse avant compactage est lavée avec la solution hydro-alcoolique. Ce lavage produit un filtrat de lavage. Cette étape optionnelle de lavage se déroule à débit de solution de lavage constant. La pression est légèrement inférieure à la pression de filtration. Les jus de sucres sont ensuite chaulés à 50°C, de la façon suivante pour atteindre un pH 10 : La concentration de la solution de lait de chaux ajoutée est égale à 15% poids. Le mélange du lait de chaux et du jus est laissé sous agitation pendant 30 à 60 minutes. Une fois le lait mélangé au jus de sucres, la chaux réagit immédiatement avec les composants non-sucrés grâce à la présence d'ions calcium et d'ions d'hydroxydes. Les ions calcium aident à la dissociation de certains acides organiques en formant les sels de calcium qui précipitent : les sels d'acide oxalique, citrique, tartrique, phosphorique. Le calcium induit aussi des réactions de coagulation de composés colorants. Les ions hydroxydes neutralisent les acides et conduisent à la précipitation d'hydroxydes de magnésium ou de fer. La concentration en ions est déterminée par chromatographie ionique, avant et après chaulage, pour les différents exemples. Elle permet de déterminer un taux d'élimination des ions sulfates, qui est donné dans le tableau 1 ci-après. Une perte en sucres de 10% est constatée après chaulage.

Les jus de sucres sont ensuite concentrés à l'aide d'un évaporateur rotatif opéré sous vide, à un niveau de concentration d'environ 300 g sucres/ kg eau.

Par ailleurs, on réalise une analyse par chromatographie gazeuse pour déterminer la teneur en éthanol (et par différence la teneur en eau) dans le solvant évaporé. Sur cette base, on réalise un calcul de l'enthalpie de vaporisation correspondant à l'énergie à apporter pour concentrer les sucres, par rapport au cas de référence (c'est-à-dire en l'absence d'éthanol dans la solution d'extraction) qui est l'exemple 1. Les gains énergétiques sont reportés dans le tableau ci-dessous.

**Tableau 1**

| Exemple | %pds Ethanol dans la solution d'extraction | %pds Ethanol dans le jus avant chaulage et évaporation | Taux d'élimination des sulfates (%) | Concentration à 300 g / kg eau | Réduction d'enthalpie de vaporisation (%) par rapport à l'exemple 1 |
|---|---|---|---|---|---|
| 1 | 0 | 0 | 86,4 | Précipitation des sels | 0,0 |
| 2 | 15 | 10 | 95,9 | Précipitation des sels | 11,3 |
| 3 | 30 | 20 | 98,2 | - | 22,6 |
| 4 | 50 | 33 | 99,7 | - | 37,7 |
| 5 | 70 | 47 | 99,9 | - | 52,8 |

Pour les exemples 1 et 2 comparatifs, où les taux d'élimination des sels sont plus faibles, on observe une précipitation des sels, qui n'est pas souhaitable (encrassement des équipements).

On voit que cette précipitation est corrélée à la teneur en éthanol dans le jus, elle-même corrélée à la teneur en éthanol de la solution d'extraction et des caractéristiques de la biomasse ici utilisée (de sa teneur initiale en eau avant extraction). La valeur minimale d'éthanol dans le jus préférée est ici d'au moins 15%poids, de préférence d'au moins 18 ou 20% poids, ce qui revient à choisir de préférence une teneur en éthanol dans la solution d'extraction supérieure à 15% poids, notamment d'au moins 18 % ou d'au moins 20% ou d'au moins 25% poids.

A noter que selon la mise en œuvre de l'extraction, notamment selon le choix de la composition (du ou des alcools) de la solution d'extraction et selon la nature de la biomasse, la teneur minimale en alcool de la solution d'extraction peut être inférieure ou égale à 15% poids, par exemple entre 5 et 15% ou entre 5 et 10% poids, et amener déjà un effet positif selon l'invention (gain énergétique et pas /peu d'encrassement par précipitation des sels). L'invention apporte un gain, selon les cas, dès que la solution d'extraction comprend une teneur en alcool non négligeable, notamment d'au moins 5% poids.

On constate de ces résultats que les exemples selon l'invention 3 à 5 avec un repulpage de la biomasse prétraitée avec une solution hydro-alcoolique permettent, à rendement d'extraction de sucres analogue dans le jus, (rendement d'extraction de 75,6% en poids pour les exemples selon l'invention) un traitement d'appauvrissement en sels minéraux du jus bien plus efficace, en passant à une élimination des sulfates de plus de 95% et même de plus de 98%, ce qui est remarquable.

En outre, on constate une forte réduction de l'enthalpie de vaporisation, d'autant plus grande que la teneur en alcool de la solution d'extraction est grande, se traduisant par une diminution du coût énergétique de l'extraction, l'évaporation d'un mélange eau-alcool étant moins consommatrice que l'évaporation d'une phase entièrement aqueuse. On peut aussi prévoir un impact favorable sur les coûts d'équipements.

## Revendications

1. Procédé de traitement d'une biomasse lignocellulosique (1), **caractérisé en ce que** le procédé comprend : - un prétraitement préalable de la biomasse par imprégnation avec une solution aqueuse acide, basique ou oxydante, puis cuisson ou explosion à la vapeur, puis - l'extraction des sucres libres de ladite biomasse prétraitée par une solution d'extraction alcoolique ou hydro-alcoolique (2) de façon à obtenir une phase liquide enrichie en sucres libres, appelée jus (3), et une phase solide appauvrie en sucres libres, appelée marc (4).

2. Procédé selon la revendication 1, **caractérisé en ce que** l'extraction est opérée à une température inférieure ou égale à 100°C, notamment inféirieure à 80°C.

3. Procédé selon l'une des revendications précédentes, **caractérisé en ce que** l'extraction des sucres est réalisée sur la biomasse prétraitée (1) présentant un taux de matière sèche MS d'au moins 25%, notamment d'au moins 30% ou d'au moins 35%, et de préférence d'au plus 70 ou 65%, notamment compris entre 30% et 65%.

4. Procédé selon l'une des revendications précédentes, **caractérisé en ce que** l'extraction des sucres libres de la biomasse prétraitée (1) comporte un contactage de ladite biomasse avec la solution d'extraction alcoolique ou hydro-alcoolique (2), notamment une mise en suspension dans ladite solution, puis une séparation solide/liquide.

5. Procédé selon la revendication précédente, **caractérisé en ce que** l'extraction des sucres libres comporte, après la séparation solide/liquide, un lavage de la phase solide (23) obtenue par ladite séparation avec une solution de lavage (24) alcoolique ou hydro-alcoolique, le lavage étant de préférence opéré en continu en co-courant ou contre-courant.

6. Procédé selon la revendication 4 ou 5, **caractérisé en ce que** le contactage de la biomasse prétraitée (1) avec la solution d'extraction alcoolique ou hydro-alcoolique (2) est opéré par un mélangeur en continu ou en discontinu, en une ou plusieurs étapes/étages.

7. Procédé selon l'une des revendications 4 à 6, **caractérisé en ce que** la séparation solide/liquide est opérée en discontinu ou en continu, notamment par un filtre-presse ou un filtre à bande.

8. Procédé selon l'une des revendications précédentes, **caractérisé en ce que** la solution d'extraction (2) et/ou de la solution de lavage (24) quand un lavage est prévu, comprend au moins un alcool choisi parmi : méthanol, éthanol, isopropanol, n-butanol, isobutanol.

9. Procédé selon l'une des revendications précédentes, **caractérisé en ce que** la solution d'extraction (2) et/ou de la solution de lavage (24) quand un lavage est prévu, est une solution hydro-alcoolique contenant au moins 5% poids d'alcool, notamment au moins 10% poids, de préférence entre 5% poids et 96% poids, ou entre 10% poids et 85% poids, ou entre 15% poids et 50 ou 70% poids d'alcool.

10. Procédé selon l'une des revendications précédentes, **caractérisé en ce qu'**on ajuste la teneur en alcool de la solution d'extraction hydro-alcoolique (2) et le rapport du débit massique de ladite solution sur le débit massique de biomasse prétraitée (1) pour que la teneur en alcool dans le jus soit d'au plus 70% poids hors soluté, de préférence d'au plus 50% poids hors soluté, et de préférence d'au moins 10% poids hors soluté.

11. Procédé selon l'une des revendications précédentes, **caractérisé en ce qu'**on traite le jus (3) obtenu pour l'appauvrir en sels minéraux, notamment par chaulage et/ou par mise en contact avec un ou des substrat(s) actifs du type résine(s) échangeuse(s) d'ions et/ou par électrodialyse.

12. Procédé selon l'une des revendications précédentes, **caractérisé en ce qu'**on concentre en sucres le jus (3) obtenu, notamment par évaporation, éventuellement après et/ou avant un traitement d'appauvrissement en sels minéraux dudit jus.

13. Procédé selon la revendication précédente, **caractérisé en ce que** la concentration est réalisée par évaporation, avec récupération de l'alcool et/ou de l'eau pour constituer tout ou partie de l'alcool et/ou de l'eau de la solution d'extraction alcoolique ou hydro-alcoolique utilisée (2) et/ou pour la solution de lavage (24) quand un lavage est prévu.

14. Procédé selon l'une des revendications précédentes, **caractérisé en ce qu'**il comprend une hydrolyse enzymatique du marc (4) obtenu après extraction, qui est suivie de, ou concomitante à, une fermentation alcoolique, puis une distillation de la fraction liquide obtenue après fermentation, et **en ce qu'**une partie de l'alcool (7) et/ou tout ou une partie de l'eau (8) récupérés lors de la distillation est/sont utilisée(s) pour constituer tout ou une partie de la solution d'extraction alcoolique ou hydro-alcoolique (2).

15. Procédé de traitement d'une biomasse lignocellulosique, ledit procédé comprenant les étapes suivantes successives :
- imprégnation de la biomasse par une solution aqueuse acide, basique ou oxydante, pour obtenir une biomasse imprégnée,
- prétraitement de la biomasse imprégnée par cuisson ou explosion à la vapeur, pour obtenir une biomasse prétraitée (1),
- hydrolyse enzymatique de la biomasse prétraitée (1), pour obtenir une biomasse hydrolysée,
- éventuellement fermentation alcoolique de la biomasse hydrolysée,
caractérisé ce qu'on opère par une solution d'extraction alcoolique ou hydro-alcoolique (2) une extraction des sucres libres d'au moins une partie de biomasse prétraitée (1), avant son hydrolyse enzymatique.
